(19) **Europäisches Patentamt**

**European Patent Office**

**Office européen des brevets**

(11) **EP 1 563 850 A2**

(12) **EUROPEAN PATENT APPLICATION**

(43) Date of publication:
**17.08.2005 Bulletin 2005/33**

(51) Int Cl.7: **A61K 47/10**

(21) Application number: **05104045.9**

(22) Date of filing: **20.02.2001**

(84) Designated Contracting States:
**AT BE CH CY DE DK ES FI FR GB GR IE IT LI LU MC NL PT SE TR**

(30) Priority: **23.02.2000 US 184273 P**

(62) Document number(s) of the earlier application(s) in accordance with Art. 76 EPC:
**01301510.2 / 1 127 580**

(71) Applicant: **Pfizer Products Inc.**
**Groton, Connecticut 06340 (US)**

(72) Inventors:
 • **Curatolo, William, John**
  **Pfizer Global Research and**
  **Groton, CT Connecticut 06340 (US)**

 • **Foulds, George Hemenway**
  **Pfizer Global Research and**
  **Groton, CT Connecticut 06340 (US)**

(74) Representative: **Hayles, James Richard et al**
 **Pfizer Limited**
 **European Patents Department,**
 **Ramsgate Road**
 **Sandwich, Kent CT13 9NJ (GB)**

Remarks:
This application was filed on 13 - 05 - 2005 as a divisional application to the application mentioned under INID code 62.

(54) **Method of increasing the bioavailability and tissue penetration of azithromycin**

(57) The bioavailability of azithromycin can be increased by co-administering azithromycin with a p-glycoprotein (p-gp) inhibitor selected from a block copolymer of poly(propylene oxide) and poly(ethylene oxide). The azithromycin and p-gp inhibitor can be administered together in a composition or as separate components. If administered separately, they can be embodied as a kit.

**EP 1 563 850 A2**

## Description

<u>Field Of The Invention</u>

This invention relates to a method for increasing the bioavailability of azithromycin, comprising co-administering azithromycin with a p-glycoprotein (p-gp) inhibitor. The invention further relates to compositions and kits comprising azithromycin and a p-gp inhibitor.

<u>Background Of The Invention</u>

Azithromycin is the U.S.A.N. (generic) name for 9a-aza-9a-methyl-9-deoxo-9a-homoerythromycin A. It is a semisynthetic, acid-stable, azalide broad spectrum antimicrobial agent produced by inserting a methyl-substituted nitrogen in place of the 9A carbonyl group in the aglycone ring of erythromycin A. It is a well known antibiotic which is readily commercially available as a therapeutic agent of choice for remediating bacterial infections. It is disclosed, *inter alia,* in US patents 4,474,768 and 4,517,359. Azithromycin has an oral bioavailability in humans of 37%.

The elimination half-life of azithromycin in blood, and more importantly in tissues, is long enough to permit single-dose therapy by dosing the entire course of therapy (usually 1.5 gm) at once. However, azithromycin exhibits gastrointestinal side effects which can prevent dosing such a high dose to certain individuals who are sensitive to azithromycin. It is known that the gastrointestinal side effects of azithromycin are locally mediated; that is, that they are due to direct contact of the drug with the gastrointestinal tract, rather than via the circulatory system. It is also known that the incidence of gastrointestinal side effects of azithromycin is dose dependent. It is not possible to predetermine which patients will be sensitive to high doses of azithromycin.

Accordingly, it would be advantageous to have a formulation of azithromycin which increased the drug's oral bioavailability, and thus could be dosed at lower doses. An especially useful formulation could provide an entire course of therapy in a single dose, while causing minimal gastrointestinal side effects due to the lower dose. For example, a formulation which is 55.5% bioavailable could be dosed at 1 gm, and provide the same systemic exposure as currently available formulations when dosed at 1.5 g.

It would be further desirable to increase the bioavailability of azithromycin, even if the goal were not to lower the dose. By increasing systemic exposure of azithromycin, it would be possible to increase tissue drug levels, thereby increasing the tissue levels above the MIC for certain pathogens which are not currently treatable by azithromycin.

It would be further desirable to increase the brain penetration of azithromycin for the treatment of syphilis and other conditions. Certain excipients and drugs, when co-dosed with another drug, increase the oral absorption of that drug. Such excipients and drugs also have the ability to increase the brain penetration of a co-dosed drug. The excipients and drugs are thought to operate, at least in part, by inhibiting drug transport via the p-glycoprotein and MDR efflux pumps, which are found in the intestinal wall and in the blood-brain barrier. By way of further explanation it is well-known that there is a series of membrane proteins called Multi-Drug Resistance (MDR) proteins, which are heavily expressed in tumor cells, and are able to excrete (or "pump") certain anticarcinogenic drugs out of the tumor cells. A portion of the resistance which tumors develop toward chemotherapy is believed to be due to the action of these proteins, which "pump" drugs out of tumor cells before the drugs have an opportunity to affect the cell. In general, it is believed that the drug passively partitions across the cell plasma membrane to get into the cell, and is actively transported out of the cell by MDR proteins. MDR proteins are also known as P-glycoproteins (p-gps).

P-gps are also present in many types of normal cells, including those of the blood-brain barrier and the intestinal epithelium, and the capillary endothelium of the testes and papillary dermis. See Cardon-Cardo et al., (1989), Proc. Natl. Acad. Sci. USA, 86, 695-698. Intestinal epithelial cells (IECs) are polarized cells which line the intestinal wall, providing a barrier between the gastrointestinal tract and the blood. The apical side of the IEC faces the intestinal lumen, and the basolateral side faces the portal blood. Most drugs are absorbed passively, first crossing the IEC apical cell membrane and entering the IEC interior, then crossing the basolateral cell membrane, thus exiting the cell on the basolateral side, entering the extracellular space and ultimately partitioning into the portal bloodstream. P-glycoproteins are located on the apical cell membrane of the IEC, and have the capacity to pump certain drugs out of the IEC back into the intestinal lumen. Thus it is possible that IEC p-gps may limit the absorption of certain drugs. The actual function of p-gps in IECs is unknown, but it has been speculated that their purpose is to slow or prevent oral absorption of toxins. The p-gp efflux pump belongs to the superfamily of ATP-binding cassette (ABC) membrane transport proteins.

P-glycoproteins exhibit low substrate specificity, and transport many kinds of molecules. The specificity is not rigorously understood, and there is no way of predicting from drug molecular structure whether a specific drug will be a substrate for intestinal p-gps. Thus It is generally not possible to predict whether a particular drug or compound will be subject to the efflux pumping action discussed above. Also, if a particular drug has low oral bioavailability, it is generally not possible to predict (1) whether the low bioavailability is caused, wholly or partially, by the efflux pumps discussed above, nor (2) whether the low bioavailability can be increased by co-administration of a p-gp inhibitor. It is unknown in the art whether the bioavailability of azithromycin can be improved by co-dosing azithromycin with another agent.

WO-95/20980 broadly claims, *inter alia,* a method for increasing bioavailabilty of an orally administered hydrophobic pharmaceutical compound, which comprises orally administering said pharmaceutical compound to a mammal in need of treatment with said compound concurrently with a bioenhancer comprising an inhibitor of a cytochrome P450-3A enzyme or an inhibitor of P-glycoprotein-mediated membrane transport. In an oral presentation at the 1996 meeting of the Controlled Release Society (Kyoto, Japan) applicant disclosed that, in cultured CACO-2 (colon carcinoma) cells, the basolateral-to-apical azithromycin flux exceeded the apical-to-basolateral flux.

Summary Of the Invention

This invention provides a method for increasing the bioavailability of azithromycin, comprising co-administering to a mammal, especially a human, in need of such treatment, a combination of azithromycin and a p-gp inhibitor. The p-gp inhibitor is administered in an amount such that the bioavailability of azithromycin is increased in comparison with what the bioavailability would be in the absence of the p-gp inhibitor (e.g., 37% when administered orally to humans). The p-gp inhibitor and azithromycin are preferably each co-administered in an amount such that the combination is antimicrobially effective.

The invention further provides a method for increasing the concentration of azithromycin in certain tissues (for example, increasing the tissue concentration effected by a given dose of azithromycin), including the brain, spinal cord, testes, and papillary dermis, comprising co-administering to a mammal, especially a human, in need of such treatment a combination of azithromycin and a p-gp inhibitor. The p-gp inhibitor is administered in an amount such that the concentration of azithromycin is increased in a tissue of interest in comparison with its concentration (i.e., effected by the same dose of azithromycin) in the absence of the p-gp inhibitor. Preferably, the p-gp inhibitor and azithromycin are each co-administered in an amount such that the combination is antimicrobially effective.

Azithromycin can be employed in this invention in the form of its pharmaceutically acceptable salts, and also in anhydrous as well as hydrated forms. All such forms are useful within the scope of the invention. The azithromycin employed is preferably the dihydrate, disclosed for example in published European Application 0 298 650 A2. Reference to "azithromycin" in terms of therapeutic amounts is to active azithromycin, i.e., the non-salt, non-hydrated macrolide molecule having a molecular weight of 749.

Use of the term "p-gp inhibitor" shall be understood to include the types of pharmaceutical and excipient compounds which are known in the art as p-gp inhibitors or as MDR inhibitors. The term p-gp inhibitor shall be understood to mean that more than one p-gp inhibitor, separately or together in a composition, can be co-administered with azithromycin. For description of the current invention, the terms p-gp and MDR are interchangeable, and include the totality of IEC and brain endothelial cell membrane pump proteins which expel drugs from these cells.

In addition, it is noted that the affinity of azithromycin for the efflux pump protein(s) in the intestinal wall is unknown, and that such affinity is generally unknown for other drugs which are inhibitors and/or are effluxed themselves. A PGP/MDR inhibitor which enhances azithromycin bioavailability or $C_{max}$ may operate by one or more of a variety of mechanisms. That is, as is well known in the art, it may be a competitive inhibitor, a non-competitive inhibitor, an uncompetitive inhibitor, or operate by a mixed mechanism. Whether such an inhibitor can affect azithromycin efflux depends, *inter alia*, upon (1) the relative affinities of azithromycin and the inhibitor for PGP/MDR, (2) the relative aqueous solubilities of azithromycin and the inhibitor, because this will affect the concentration of the two at the pump in vivo when they are in competition, (3) the absolute aqueous solubility of the inhibitor, because it must achieve a sufficient concentration at the pump in vivo to effectively inhibit the pump, and (4) the dose of the inhibitor. For the purpose of this invention, a "PGP/MDR inhibitor" is any compound which improves the systemic exposure of azithromycin, when azithromycin is dosed orally or by any other route, and which is effluxed by and/or inhibits one or more of the drug efflux proteins/activities of intestinal epithelial cells. Evidence of efflux and/or inhibition may be obtained in an *in vitro* test such as a test of competition with, or inhibition of, azithromycin efflux in a cell culture model for intestinal epithelial cells. The Caco-2 cell model is one such IEC model. Likewise, blood-brain barrier efflux may be determined using cultured brain endothelium cells. See Begley, (1996), J. Pharm. Pharmacol., 48, 136-146. This definition of "PGP/MDR inhibitor" applies to any "PGP/MDR inhibitor" of this invention, whether or not the "PGP/MDR inhibitor" is a drug.

Reference to "administration", "administering", "dosage" and "dosing" includes administration by any route unless a particular route is specified.

"Co-administration" of a combination of azithromycin and a p-gp inhibitor means that the two components can be administered together as a composition or as part of the same, unitary dosage form. Co-administration also includes administering azithromycin and a p-gp inhibitor separately but as part of the same therapeutic regimen. The two components, if administered separately, need not necessarily be administered at essentially the same time, although they can if so desired. Thus co-administration includes, for example, administering azithromycin plus a p-gp inhibitor as separate dosages or dosage forms, but at the same time. Co-administration also includes separate administration at different times and in any order

In this respect, azithromycin is unusual because it has a very long elimination half-life (69 hr), and is a rare drug because it is known to undergo significant transintestinal elimination. For example, in the dog 40% of the recovered radiolabel from an intravenous radiolabeled azithromycin dose is eliminated across the intestinal wall, and this would also be true of elimination of an oral dose (Foulds et al, 1991, Program and Abstracts of the 5th European Congress of Clin. Microbiology and Infectious Diseases, Oslo, Norway). In humans, it has also been demonstrated that azithromycin is eliminated into the intestine, although the relative contribution of biliary and transintestinal elimination has not been determined (Luke and Foulds, 1997, Clin. Pharmacol, Ther. 61, 641-648).

Thus a single dose of, say, 1 gm azithromycin may be co-dosed, e.g. orally, with a p-gp inhibitor to increase azithromycin absorption, and then subsequent dosing of the p-gp inhibitor over the next week will inhibit transintestinal elimination of azithromycin, thus maintaining higher serum and tissue levels of azithromycin. For example, azithromycin may be dosed as a single dose with nelfinavir, and continued dosing with nelfinavir (usually dosed three times daily) will have a beneficial effect on azithromycin therapy.

It should be noted that a variety of azithromycin regimens are currently used therapeutically. Currently used dose regimens include (1) 500 mg once daily for 3 days; (2) 500 mg dosed once on day 1, followed by 250 mg dosed once on days 2, 3, 4, 5; (3) 1 gm dosed once; (4) 1200 mg dosed once per week for a year or more. Other azithromycin dose regimens are possible.

From the above discussion, those skilled in the art will appreciate that a variety of azithromycin/p-gp inhibitor dose regimens are possible. Useful dose regimens are those which increase the bioavailability or $C_{max}$ or brain penetration or other tissue levels of azithromycin.

In a preferred embodiment, azithromycin and a p-gp inhibitor are dosed at the same time, i.e., within 15 min of each other. In a more preferred embodiment, azithromycin and p-gp inhibitor are dosed at the same time, i.e., within 15 min of each other, and the p-gp inhibitor is dosed on subsequent days for up to 2 weeks or even longer.

In a preferred embodiment, a p-gp inhibitor is co-administered in an amount or regimen such that the oral bioavailability of azithromycin is increased by at least 25% (i.e., to an absolute oral bioavailability of at least 46%). Oral bioavailability can be assessed as known in the art by measuring AUCs, where AUC is the area under the curve (AUC) plotting the serum or plasma concentration of drug along the ordinate (Y-axis) against time along the abscissa (X-axis). Generally, the values for AUC represent a number of values taken from all the subjects in a patient test population and are, therefore, mean values averaged over the entire test population.

In a more preferred embodiment, a p-gp inhibitor is co-administered in an amount or regimen such that the oral bioavailability of azithromycin is increased by at least 50% (i.e., to an absolute oral bioavailability of at least 55%).

In a still more preferred embodiment, a p-gp inhibitor is co-administered in an amount or regimen such that the oral bioavailability of azithromycin is increased by at least 75% (i.e., to an absolute oral bioavailability of at least 65%).

Co-dosing azithromycin and a p-gp inhibitor can also increase Cmax relative to dosing azithromycin in the absence of a p-gp inhibitor, and this is provided as a further aspect of the invention. Cmax is also well understood in the art as an abbreviation for the maximum drug concentration in serum or plasma (serum concentration in the case of azithromycin) of the test subject.

In a preferred embodiment, a p-gp inhibitor is co-administered in an amount or regimen such that the oral $C_{max}$ of azithromycin is increased by at least 25% compared to dosing in the absence of a p-gp inhibitor.

In a more preferred embodiment, a p-gp inhibitor is co-administered in an amount or regimen such that the oral $C_{max}$ of azithromycin is increased by at least 50% compared to dosing in the absence of a p-gp inhibitor.

In a still more preferred embodiment, a p-gp inhibitor is co-administered in an amount or regimen such that the oral $C_{max}$ of azithromycin is increased by at least 75% compared to dosing in the absence of a p-gp inhibitor.

The concentration of azithromycin in tissues, e.g. brain, testes, may be determined by standard methods, such as those described by Foulds et al. (1990), J. Antimicrob. Chemotherapy, 25, Suppl. A, 73-82.

Embodiments of this invention include regimens and combinations of p-gp inhibitors and azithromycin which increase the concentration of azithromycin in any mammalian tissue, e.g. brain, or cell type, e.g. macrophage, at any time post-dose.

In a preferred embodiment, a p-gp inhibitor is co-administered in an amount or regimen such that the concentration of azithromycin in a tissue or cell is increased by at least 25% (i.e., to at least 1.25-fold the concentration in the absence of p-gp dosing), at any time post-dose.

In a more preferred embodiment, a p-gp inhibitor is co-administered in an amount or regimen such that the concentration of azithromycin in a tissue or cell is increased by at least 50% (i.e., to at least 1.5-fold the concentration in the absence of p-gp dosing), at any time post-dose.

In a still more preferred embodiment, a p-gp inhibitor is co-administered in an amount or regimen such that the concentration of azithromycin in a tissue or cell is increased by at least 75% (i.e., to at least 1.75-fold the concentration in the absence of p-gp dosing), at any time post-dose.

In a preferred embodiment, the p-gp inhibitor is an antimalarial drug such as chloroquine, hydroxychloroquine, quinidine, or quinine; an anti-AIDs drug such as nelfinavir, saquinavir, ritonavir, and indinavir; an antibiolic such as cefoperazone and ceftriaxone; an antifungal such as itraconazole; an immunosuppressant such as cyclosporine; a calcium channel blocker such as verapamil.

In a preferred embodiment, the p-gp inhibitor is a non-pharmaceutical selected from the class of polymers which are block copolymers of poly(propylene oxide) and poly(ethylene oxide). Examples of these block copolymers are available under the registered trademark PLURONIC® from BASF. Preferred polymers include PLURONIC L43, L61, L62, L64, L81, L92, L101, P85, P103, P104, P123, all available from BASF Corp., Parsippany, NJ. More preferred are Pluronic L61, L62, L64, L81, L92, P85, P103, P104.

In a preferred embodiment, the p-gp inhibitor is a surfactant selected from the class of non-ionic surfactants. Examples are the PEO esters of oleic acid, preferably those wherein the PEO content is in the range 20-30 PEO units per molecule. Examples also include PEO esters of stearic acid, preferably those including 10-35 PEO units per molecule. Useful surfactant p-gp inhibitors include polyoxyethylene ethers (e.g. Brij series), p-t-octylphenoxypolyoxyethylenes (e.g. Triton X-100), nonylphenoxypolyoxyethylenes (e.g. Igepal CO series), polyoxyethylene sorbitan esters (e. g. Tween series, such as polysorbate 80), ethoxylated fatty acids (e.g. Myrj series), polyoxyethyleneglycerides (e.g. Gelucire series, such as Gelucire 44/14), and sucrose fatty acid esters (e.g. Ryoto sugar ester series).

More preferred non-ionic surfactant p-gp inhibitors have a hydrophile-lipophile balance (HLB) number greater than about 13.

Compositions comprising azithromycin and a p-gp inhibitor are provided as an additional feature of the invention.

Since the present invention has an aspect that relates to treatment with a combination of compounds which may be co-administered separately, the invention also relates to combining separate pharmaceutical compositions in kit form. The kit comprises two separate pharmaceutical compositions: (1) a composition comprising azithromycin, plus a pharmaceutically acceptable carrier or diluent; and (2) a composition comprising a p-gp inhibitor, plus a pharmaceutically acceptable carrier or diluent. The amounts of (1) and (2) are such that, when co-administered separately, the bioavailability of azithromycin is increased. The kit comprises a container for containing the separate compositions such as a divided bottle or a divided foil packet, wherein each compartment contains a plurality of dosage forms (e.g., tablets) comprising (1) or (2). Alternatively, rather than separating the active ingredient-containing dosage forms, the kit may contain separate compartments each of which contains a whole dosage which in turn comprises separate dosage forms. An example of this type of kit is a blister pack wherein each individual blister contains two (or more) tablets, one (or more) tablet(s) comprising pharmaceutical composition (1), and the second (or more) tablet(s) comprising pharmaceutical composition (2). Typically the kit comprises directions for the administration of the separate components. The kit form is particularly advantageous when the separate components are preferably administered in different dosage forms (e.g., oral and parenteral), are administered at different dosage intervals, or when titration of the individual components of the combination is desired by the prescribing physician. In the case of the instant invention a kit therefore comprises

    (1) a therapeutically effective amount of a composition comprising azithromycin, plus a pharmaceutically acceptable carrier or diluent, in a first dosage form;
    (2) a therapeutically effective amount of a composition comprising a compound which is a p-gp inhibitor, plus a pharmaceutically acceptable carrier or diluent, in a second dosage form; and
    (3) a container for containing said first and second dosage forms.

The invention is surprising in that the published literature generally states that azithromycin exhibits surprisingly minimal drug interaction when co-dosed with other drugs. For example, as reported in Zimmerman et al. (1996) Arzneim.-Forsch., Drug Res., 46, 213-217, co-dosing azithromycin with midazolam has no effect on (i.e. does not increase) midazolam plasma levels. In the same study, midazolam was co-dosed with erythromycin, and midazolam plasma levels were greatly increased (380% increase in AUC). Backman et al., (1995), Int. J. Clin. Pharmacol. Therapeut., 33, 356-359, found that azithromycin did not increase the plasma concentrations of oral midazolam. A review by Malaty and Kuper, (1999), Drug Safety, 20, 147-169. states that azithromycin does not interact (have an effect on the plasma levels of) the HIV protease inhibitors saquinavir, ritonavir, indinavir, and nelfinavir. In the same article the macrolide antibiotic clarithromycin was stated to exhibit an interaction with all but nelfinavir.

Detailed discussion

The p-gp inhibitor, in one aspect, may be widely chosen from numerous compounds, including compounds which are non-pharmaceutical in the sense that they are not known to exhibit any therapeutic effect and/or which, but for their p-gp inhibitory activity, would be considered therapeutically inactive. The p-gp inhibitor, in a second aspect, may be selected from compounds which are themselves drugs and which, in addition to their known therapeutic function (s), also inhibit p-gp.

Examples of p-gp inhibitors which are otherwise generally considered to be excipients and/or therapeutically inactive, include the surfactants and polymers previously disclosed herein. Such therapeutically inactive p-gp inhibitors and/or excipients also include the following listed in Table 1. These inhibitors may be dosed at doses of 25 mg to 3 gm, preferably 50 mg to 2 gm, more preferably 50 mg to 1 gm.

## Table I. Excipients and Non-pharmacological Agents which Increase the Bioavailability of Azithromycin

| Excipient/Agent |
| --- |
| PPO-PEO Block copolymers (Pluronics) |
| Cremophor-EL |
| d-alpha-tocopheryl-polyethyleneglycol-1000-succinate |
| Solutol-HS-15 |
| Polysorbate-80 |
| Oleic acid PEO esters |
| Stearic acid PEO esters |
| Triton-X100 |
| Nonidet P-40 |
| Benzoin gum |

Examples of p-gp inhibitors which are drugs having a therapeutic function other than p-gp inhibition include the following drugs listed in Table 2:

Table II.

| Drugs and Drug Analogues which Increase the Bioavailability of Azithromycin | |
| --- | --- |
| Drug | Drug |
| Amiodarone | Aldosterone |
| Lidocaine | Clomiphene |
| Cefoperazone | Cortisol |
| Ceftriaxone | Dexamethasone |
| Erythromycin | Prednisone |
| Itraconazole | Progesterone |
| Chloroquine | Tamoxifen |
| Emetine | Desipramine |
| Quinidine | Trazodone |

Table II.   (continued)

| Drugs and Drug Analogues which Increase the Bioavailability of Azithromycin ||
| Drug | Drug |
| --- | --- |
| Hydroxychloroquine | Dipyridamole |
| Quinacrine | Reserpine |
| Quinine | Cyclosporin A |
| Bepridil | Colchicine |
| Diltiazem | FK-506 |
| Felodipine | Quercetin |
| Nifedipine | SDZ PSC-833 |
| Nisoldipine | SDZ 280-446 |
| Nitrendipine | Terfenadine |
| Tiapamil | Tumor Necrosis Factor |
| Verapamil | Vitamin A |
| Actinomycin D | Etoposide |
| Daunorubicin | R-Verapamil |
| Mitomycin-C | Ketoconazole |
| Taxol | Tamoxifen |
| Trimetrexase | RU-486 |
| Vinblastine | Devapamil |
| Vincristine | Gallopamil |
| Indinavir | Emopamil |
| Nelfinavir | L-Emopamil |
| Saquinavir | R-Emopamil |
| Ritonavir | L-Verapamil |
| Bupivacaine | Phenothiazines |

The drugs listed in Table 2 can be administered in their conventional dosage amounts, as known in the art, for example from the Physician's Desk Reference.

A particularly preferred group of p-gp inhibitors for use in this invention includes the following:

nelfinavir
saquinavir
ritonavir
indinavir
verapamil
cefoperazone
ceftriaxone
cyclosporine
chloroquine
quinidine
hydroxychloroquine
quinine

As stated previously, the invention can be embodied as a kit. An example of a kit, as alluded to previously, is a so-called blister pack. Blister packs are well known in the packaging industry and are widely used for the packaging of pharmaceutical unit dosage forms such as tablets, capsules, and the like. Blister packs generally consist of a sheet of relatively stiff material covered with a foil of a preferably transparent plastic material. During the packaging process recesses are formed in the plastic foil. The recesses have the size and shape of the tablets or capsules to be packed. Next, the tablets or capsules are placed in the recesses and the sheet of relatively stiff material is sealed against the plastic foil at the face of the foil which is opposite from the direction in which the recesses were formed. As a result, the tablets or capsules are sealed in the recesses between the plastic foil and the sheet. Preferably, the strength of the sheet is such that the tablets or capsules can be removed from the blister pack by manually applying pressure on the recesses whereby an opening is formed in the sheet at the place of the recess. Tablet(s) or capsule(s) can then be removed via said opening.

It may be desirable to provide a memory aid on the kit, e.g., in the form of numbers next to the tablets or capsules whereby the numbers correspond with the days of the regimen during which the tablets or capsules so specified should be ingested. Another example of such a memory aid is a calendar printed on the card, e.g., as follows "First Week, Monday, Tuesday, ...etc.... Second Week, Monday, Tuesday,...", etc. Other variations of memory aids will be readily apparent. A "daily dose" can be a single tablet or capsule or several pills or capsules to be taken on a given day. Also a daily dose of the first compound can consist of one tablet or capsule while a daily dose of the second compound can consist of several tablets or capsules and vice versa. The memory aid should reflect this.

In general, the azithromycin will be dosed at (1) the same level it would be dosed in the absence of a particular p-gp inhibitor if the goal is to increase the intracellular level of azithromycin; (2) a decreased level relative to the normal level it would be dosed at in the absence of the p-gp inhibitor. The dose of azithromycin in the second case will usually be the normal dose proportionately decreased according to the increased bioavailability. For example, if the bioavailability in the presence of p-gp inhibitor is 50%, then a 1 gm dose may be decreased to 1 gm x 37/50=0.74 gm, where 37% is the non-enhanced oral bioavailability of azithromycin. The azithromycin may also be administered at an intermediate level between the two dosage values. In general, azithromycin will be administered orally in an amount of from 25 to 3000 mg per dose, preferably 100 to 2000 mg per dose, in a single or divided dosage form. If administered separately from the p-gp inhibitor, any oral dosage form of azithromycin, including suspensions, tablets, capsules, and unit dose packets (referred to in the art as "sachets") can be employed, as known in the art, for example from the latest Physicians Desk Reference.

While azithromycin and p-gp inhibitor may both be dosed orally, either or both may also be dosed by another route. For example, azithromycin may be dosed intravenously, and the p-gp inhibitor may be dosed intravenously or orally. Because the p-gp inhibitor will inhibit transintestinal elimination of intravenously-dosed azithromycin, it will maintain azithromycin in the body (i.e. in the systemic circulation and in tissues) for longer than would occur in the absence of p-gp inhibitor.

In a preferred embodiment, when azithromycin is dosed intravenously, a p-gp inhibitor is co-administered, orally or intravenously, in an amount or regimen such that the serum azithromycin AUC is increased by at least 25%, i.e. 1.25-fold the AUC in the absence of p-gp inhibitor.

In a more preferred embodiment, when azithromycin is dosed intravenously, a p-gp inhibitor is co-administered, orally or intravenously, in an amount or regimen such that the serum azithromycin AUC is increased by at least 50%.

In a still more preferred embodiment, when azithromycin is dosed intravenously, a p-gp inhibitor is co-administered, orally or intravenously, in an amount or regimen such that the serum azithromycin AUC is increased by at least 75%.

For the purpose of increasing the brain penetration of azithromycin, an orally dosed p-gp inhibitor must be orally absorbed. Alternatively, the p-gp inhibitor may be dosed intravenously, subcutaneously, intramuscularly, or intrathecally.

As previously disclosed, the combination of azithromycin and p-gp inhibitor can be administered as a composition. The components can be administered together in any conventional oral dosage form, usually also together with a pharmaceutically acceptable carrier or diluent.

For oral administration the pharmaceutical composition comprising azithromycin and p-gp inhibitor can take the form of solutions, suspensions, tablets, pills, capsules, powders, unit dose packets and the like. Tablets containing various excipients such as sodium citrate, calcium carbonate and calcium phosphate can be employed along with various disintegrants such as starch and preferably potato or tapioca starch and certain complex silicates and microcrystalline cellulose, together with binding agents such as polyvinylpyrrolidone, sucrose, gelatin and acacia. Additionally, lubricating agents such as magnesium stearate, sodium lauryl sulfate and talc are often very useful for tabletting purposes. Solid compositions of a similar type are also employed as fillers in hard-filled gelatin capsules; preferred materials in this connection also include lactose or milk sugar as well as high molecular weight polyethylene glycols. When aqueous suspensions and/or elixirs are desired for oral administration, the compounds of this invention can be combined with various sweetening agents, flavoring agents, coloring agents, emulsifying agents and/or suspending agents, as well as such diluents as water, ethanol, propylene glycol, glycerin and various like combinations thereof. If the compositions are embodied as a suspension or a unit dose packet, they can be formulated in the same manner and contain the same excipients as known for use in formulations of azithromycin alone. If the dosage form is a suspension, it will be common to include one or more thickening agents, a dispersing agent, and a buffer or pH-altering agent. If the dosage form is a unit dose packet, it will generally also contain a dispersing agent. Solutions or suspensions of azithromycin in a vehicle, such as polyethylene glycol-400 or a glyceride oil, may be encapsulated in soft gelatin capsules.

The types of ingredients which can be included in different types of azithromycin formulations are disclosed, for example, in US 5,605,889, herein incorporated by reference.

The efficacy of a compound (drug, non-drug, or otherwise) as a p-gp inhibitor can be shown and approximated by a CACO-2 cell assay as described, for example, in Kim et al. (1998) J. Clin. Invest. 101, 289-294., and also in the examples below. Caco-2 cells are colon carcinoma cells which are considered in the art to be a reasonable model for the intestinal epithelium. The ability of a compound to inhibit p-gp/MDR-facilitated azithromycin efflux may be determined in a Caco-2 cell assay. However, improved azithromycin bioavailability is best shown by human clinical studies, of the type illustrated in the Examples.

The invention is further disclosed and described by means of the following non-limiting examples

EXAMPLE 1. Azithromycin Transport Across Caco-2 Cell Monolayers.

Caco-2 cell monolayers were grown on permeable transwell-col filter supports (24.5mm diameter, 0.45µm pore size) and used in these studies at day 21-24. 2 mls of 0.4 µM $^{14}$C Azithromycin in Hank's balanced salt solution (HBSS) was added to the donor chamber and 2mls of HBSS was added to the acceptor chamber. An impermeable marker ,$^{3}$H Mannitol, was included in these studies to ensure the monolayers were intact. Monolayers exhibiting >1% mannitol flux/hr were disregarded from the study. Transport was monitored in the apical to basolateral (absorptive) and basolateral to apical (secretory) direction. The wells were incubated for 1 hour (unstirred) at 37°C. Samples were removed after the incubation period and analyzed by dual label LSC. The transport was calculated as percent flux/hr.

$$\% \text{ Flux/hr} = \frac{\text{amount transported}}{\text{total amount recovered from donor and acceptor chamber}} \times 100$$

Where P-glycoprotein inhibitors, verapamil and [4-(6,7-dimethoxy-3,4-dihydro-1 H-isoquinolin-2-yl)-6,7-dimethoxyquinazolin-2-yl]-[2-(3,4-dimethoxyphenyl)-ethyl]-amine (Inhibitor A) were included, they were added to both the donor and acceptor chambers at concentrations of 0.2mM and 40 µm, respectively. Transport measurements were made in controls, implemented in like fashion except no test compound was included.

The results are given in the following table.

|  | % flux/hr A-B | St.Dev | % Flux/hr B-A | St.Dev |
|---|---|---|---|---|
| Control (no inhibitor) | 0.1 | 0.06 | 6.6 | 0.8 |
| 0.2mM Verapamil | 0.3 (n=2) | 0.07 | 0.5 | 0.09 |
| 40 µm Inhibitor A | 0.7 | 0.1 | 0.6 | 0.05 |
| 'n' >or = to n=3 unless stated otherwise | | | | |
| N.B. Values of less than 1 % are not accurate due to the assay sensitivity. | | | | |

These data demonstrate that azithromycin is a substrate for the P-glycoprotein efflux transporter. Azithromycin exhibits a much higher secretory flux (basolateral-to-apical; B-A) than absorptive flux (apical-to-basolateral; A-B). The basolateral-to-apical transport of azithromycin is inhibited by the P-glycoprotein inhibitors verapamil and Inhibitor A. In the presence of these inhibitors, the apical-to-basolateral azithromycin flux is clearly increased; however the absolute flux values are low and thus not numerically accurate.

EXAMPLE 2. Effect of Nelfinavir on the Pharmacokinetics of Azithromycin

This was an open-label, randomized, two-way, two-treatment, crossover design study of the effect of nelfinavir (Viracept®, registered trademark of Agouron Pharmaceuticals, Inc.) on the pharmacokinetics of a single 1200 mg oral dose of azithromycin, at nelfinavir steady state in normal subjects. Healthy volunteers received nelfinavir for 11 days. On Day 9 a single dose of azithromycin was administered. Each subject also received a single control dose of 1200 mg azithromycin either two weeks before the start of a nelfinavir treatment regimen or three weeks after a nelfinavir treatment regimen.

Nelfinavir was administered as 3 commercial 250 mg tablets, 3 times per day (morning dose at approximately 7 am; afternoon dose at approximately 3 pm; evening dose at approximately 10 pm) with food for 11 days. On day 9, azithromycin (1200 mg) was dosed as 2 600 mg commercial tablets at the same time as the morning dose of nelfinavir. On day 9 the subjects, who were previously fasted for at least 8 hr, consumed a standard breakfast consisting of cereal and/or toast with butter and jelly, and milk. Immediately following the standard breakfast, the subjects consumed 750 mg nelfinavir and 1200 mg azithromycin, with 120 ml water. On the day on which azithromycin alone was dosed, 2 600 mg commercial tablets were dosed with 120 ml water, immediately following the standard breakfast.

Serum azithromycin concentrations were determined pre-dose, and at 1, 2, 3, 4, 6, 8, 12, 24, 48, 72, 96, 120, 144, and 168 hr post-dose.

Serum samples were assayed for azithromycin utilizing LC/MS/MS. The azithromycin assay had a dynamic range of 10.4 to 1000 ng/ml. Concentrations below the lower limits of quantification were utilized as 0.00 μg/ml in the calculations.

Maximum observed azithromycin concentrations (Cmax) were determined by inspection of the data. Tmax was defined as the time of first occurrence of Cmax. Area under the serum concentration versus time curves ($AUC_{0-168}$) were calculated for the interval of pre-dose to 168 hours post-dose. Area under the serum concentration versus time curves ($AUC_{last}$) were calculated for the interval of pre-dose to the last time at which concentrations of azithromycin were measurable. Total exposure was estimated as AUC for the interval of pre-dose to infinity. $AUC = AUC_{last} + C^{*}_{last}/k_{el}$, where $C^{*}_{last}$ is the concentration estimated from the aforementioned regression at the time of the last quantifiable concentration of drug.

Geometric mean values of Cmax, AUC, Cmax ratios, and AUC ratios were determined. Arithmetic means of other parameters were determined.

Mean $AUC_{0-\infty}$ for azithromycin increased 112% (90% confidence interval = 180% to 250%, p = 0.0001) from 11.5 μg.hr/ml to 24.5 μg.hr/ml following co-administration with nelfinavir. Mean Cmax increased by 137% (90% confidence interval = 177% to 315%; p = 0.0003) from 889 ng/ml to 2100 ng/ml following co-administration with nelfinavir.

## Claims

1. Use of a p-gp inhibitor in the manufacture of a medicament for having increased bioavailability of orally or intravenously-administered azithromycin, wherein said p-gp inhibitor is selected from block co-polymers of poly(propylene oxide) and poly(ethylene oxide).

2. The use as defined in claim 1, wherein said azithromycin and p-gp inhibitor are each administered in an amount such that the combination is antimicrobially effective.

3. The use as defined in claim 1, wherein said bioavailability increase is measured in blood serum.

4. The use as defined in claim 1, wherein said p-gp inhibitor and azithromycin are co-administered separately.

5. The use as defined in claim 4, wherein said p-gp inhibitor and azithromycin are co-administered by different routes.

6. The use as defined in claim 5, wherein said p-gp inhibitor is administered orally and said azithromycin is administered intravenously.

7. The use as defined in claim 4, wherein said azithromycin and said p-gp inhibitor are both administered orally.

8. The use as defined in claim 1, wherein said p-gp inhibitor and azithromycin are co-administered together in a composition.

9. The use as defined in claim 1, wherein said p-gp inhibitor is co-administered in an amount such that the oral bioavailability of azithromycin is increased by at least 25%.

10. The use as defined in claim 1, wherein said increase is measured as an increase in AUC relative to dosing in the absence of a p-gp inhibitor.

11. The use as defined in claim 1, wherein said mammal is a human.

**12.** A composition comprising azithromycin and a p-gp inhibitor, wherein said p-gp inhibitor is selected from block co-polymers of poly(propylene oxide) and poly(ethylene oxide).

**13.** The composition as claimed in claim 12, wherein said p-gp inhibitor is present in an amount such that, following administration, the oral bioavailability of azithromycin is increased.

**14.** The composition as defined in claim 13, wherein said p-gp inhibitor is present in an amount such that said oral bioavailability of azithromycin is increased by at least 25%.

**15.** The composition as defined in claim 14, wherein said p-gp inhibitor is co-administered in an amount such that the oral bioavailability of azithromycin is increased by at least 50%.

**16.** The composition as defined in claim 15, wherein said p-gp inhibitor is co-administered in an amount such that the oral bioavailability of azithromycin is increased by at least 75%.

**17.** The composition as defined in claim 13 which increases the Cmax of azithromycin.

**18.** The composition as defined in claim 17, wherein said p-gp inhibitor is present in an amount such that said Cmax of orally or intravenously administered azithromycin is increased by at least 25%.

**19.** Pharmaceutical products containing azithromycin for oral or intravenous administration and a p-gp inhibitor as a combined preparation for simultaneous, separate or sequential use in treating a mammal in need of azithromycin administration, wherein said p-gp inhibitor is selected from block co-polymers of poly(propylene oxide) and poly(ethylene oxide).

**20.** The pharmaceutical product of claim 19, wherein said mammal in need of azithromycin administration is a mammal with a microbial infection.

**21.** The pharmaceutical product of claim 19 or claim 20, wherein the mammal is a human.